# EUROPEAN PATENT APPLICATION

(11) **EP 2 246 037 A2**
(43) Date of publication of application: **03.11.2010**
(21) Application number: 09006147.4
(22) Date of filing: 06.05.2009
(51) Int. Cl.: A61K 8/49, A61Q 19/02, A61Q 19/08, A61K 8/63, G01N 33/50

(54) **Anti-aging and whitening properties of quercetin, 18a-glycyrrhetinic acid, hederagenin and its derivatives**

(30) Priority: 27.04.2009 GR 20090100242
(71) Applicant: Korres S.A. Natural Products, 32011 Inofyta (GR)
(72) Inventor: Gonos, Eustathios, 14564 Kifisia (GR); Chinou, Ioanna, 15669 Papagou (GR); Chondrogianni, Niki, 11634 Athens (GR)
(74) Representative: Karakatsanis, Georgios

(57) **Abstract**

Quercetin, 18α-glycrrhetinic and/or hederagenin and/or its derivatives are used as anti-aging and/or whitening agents for the treatment of skin. Methods for testing the anti-aging and whitening properties of compounds are disclosed.

## Description

The invention refers to the use of natural compounds extracted from plants of the Greek flora with anti-aging properties on human diploid fibroblasts. These anti-ageing properties are mediated through the decrease of cellular oxidative load as well as through the activation and maintenance of the activity of the main proteolytic pathway of the cell and one of the central secondary anti-oxidant system, the proteasome. Furthermore, this invention refers to the use of these compounds as natural whiteners against age spots.

According to the invention quercetin, 18α-glycyrrhenitic acid and/or hederagenin and/or its derivatives are used as effective compounds in a composition for the treatment of skin having anti-aging and/or whitening properties.

According to the invention the composition may contain only one of these effective compounds or two, three or more of them. Furthermore, the composition may contain at least one of these effective compounds and at least another compound having anti-aging and/or whitening properties.

According to the invention these compound are effective against senescence of diploid fibroblasts one of the main cell type in skin that play a central rule in the tissue's aging.

Claims 2 to 7 refer to the improvement and the maintenance of the young morphology of diploid fibroblasts and whitening of melanocytes, respectively, by treatment with these compounds. In case of claim 3 the increase of the proliferation of diploid fibroblasts during senescence is limited or controllable. Claims 8 to 18 define preferred methods for testing the anti-aging and whitening properties of a certain compound.

When the morphology is examined according to claim 10 the ratio of elongated to flattened diploid fibroblast cells can be determined. A preferred oxidizing agent for the method of claim 15 is hydrogen peroxide.

**Quercetin has the formula I and its derivative the formula II.** 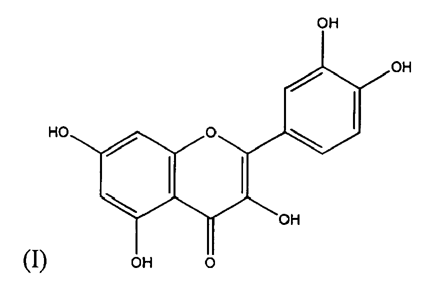 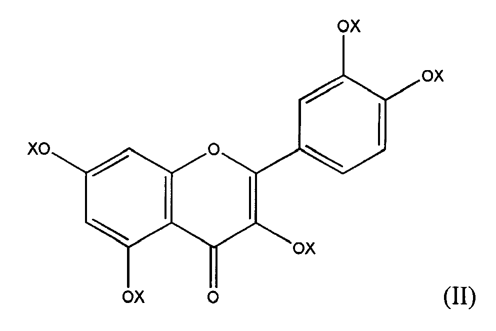 Quercetin (formula I) is the aglycon form of a number of other flavonoid (flavonol ) glycosides such as rutin (rhamnoglucoside of quecetin -rutinoside), quercitrin (glucoside of quercetin) etc.. Main quercetin and many of its derivatives, has been found to be among the most active of the flavonoids in studies and many medicinal plants owe much of their activity to their high quercetin and quercetin derivatives content. Quercetin and many of its derivatives have demonstrated significant antiinflammatory and cytotoxic activity, they also exert strong potent antioxidant activity, while extracts rich in quercetin and its derivatives may have positive effects to prevent against allergies-inflammation, heart diseases etc. Natural sources rich in quercetin and its derivatives, include Oak tree (Quercus sp.), capers (Capparis spinosa), red grapes (Vitis vinifera), apples (Pyrus malus), onions (Allium cepa), tea (Camellia sinensis), Citrus fruits (Citrus sp.), lovage, brocoli, other leafy green vegetables, cherries and many species of berries such as rasberry, bog whortleberry, chokeberry, crowberry etc. Quercetin and its derivatives have been also referred in several varieties of honeys.

The invention comprises the use of quercetin (formula I) as well as of quercetin derivatives according to formula II in which a hydrogen atom of at least one hydroxyl group of quercetin is subsituted by another group. Preferred quercetin derivatives are quercetin caprylate and quercetin glycosides. In quercetin caprylate any hydroxyl group in an position (1, 2, 3, 4, 5) may be substituted with caprylic acid. That means any X in formula (II) may be an caprlyic group. In quercetin glycosides quercetin is substituted in position 3 with sugar. The sugar may be for instance a monosaccharide as disaccharide as rutinose in case of rutin.

### 18α-glycyrrhetinic acid has the formula II and its derivative the formula IV.

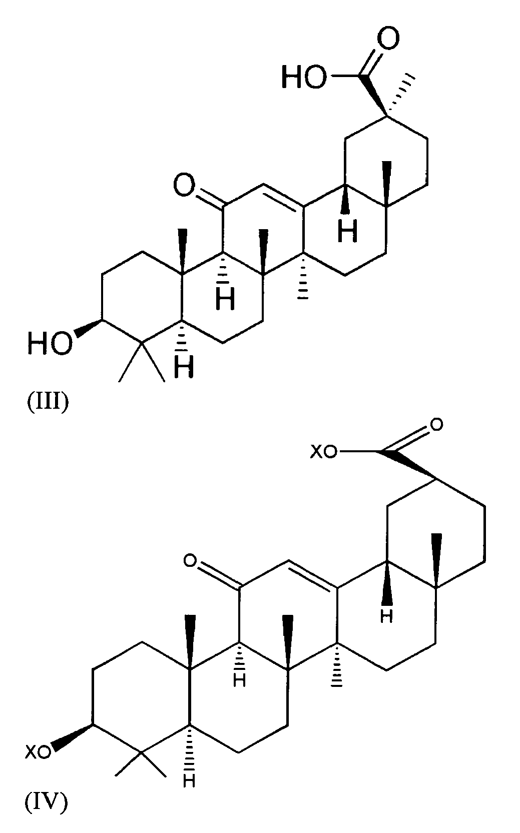

**18**α**-glycyrrhetinic acid** (formula III) is a triterpene mainly occurring in nature from ***Glycyrrhiza.** Glycyrrhiza* is a genus of about 18 accepted species in the family Fabaceae (Leguminosae), with a subcosmopolitan distribution in Asia, Australia, Europe and America (North and South). The genus is best known for liquirice, which is the product of *Glycyrrhiza glabra,* a species native to the Mediterranean region and very common and widespread in Greece.

The invention comprises the use of 18α-glycyrrhetinic acid (formula III) as well as of its derivatives according to formula IV in which a hydrogen atom of the hydroxyl group and/or the carboxyl group of 18a-glycyrrhetinic acid is substitued by another group as a sugar.

### Hederagenin has the formula V and its derivative the formula VI.

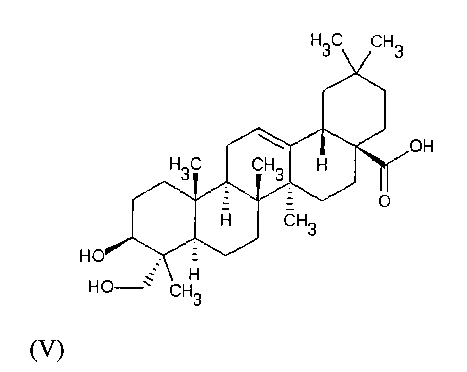

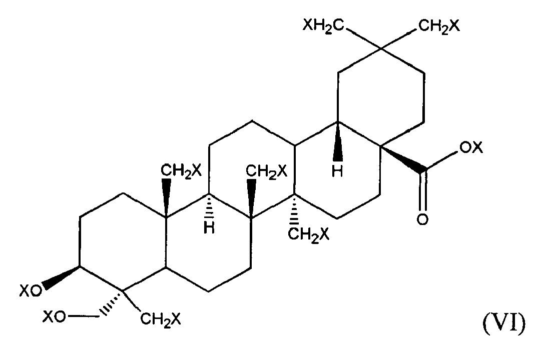

Hederagenin is a triterpenic saponin (formula V) which can be found widespread in nature in many plant species but mainly in the leaves of Ivy (*Hedera helix* L., Araliaceae), which is an evergreen medicinal and ornamental and from which it has also taken its name. Such triterpene and similar derivatives can be determined and isolated from the stems of *Humulus lupulus* L. (Hops), from the plant of Medicago sativa subsp. sativa (Alafalfa), as well from the seeds of *Nigella sativa* L.; (Black Caraway, Black Cumin) and the roots of *Paeonia* species (such as White Peony *Paeonia lactiflora* PALL.) and *Pulsatilla* sp. (such as *Pulsatilla chinensis* ).

The invention comprises the use of hederagenin (formula V) as well as its derivatives according to formula VI in which a hydrogen atom of at least one hydroxyl group and/or at least one methyl group and/or the carboxyl group of hederagenin is substituted by onaother group as a sugar.

Anti-aging properties of the examined substances in human diploid fibroblasts were evaluated by the following biomarkers: 1) Cellular lifespan in the presence of the substances as compared to the lifespan performed by control cultures (diluent-treated) and morphology of the cells following treatment with the substances throughout their lifespan. 2) Detection of β-galactosidase activity. 3) Reversal of senescent phenotype by addition of the substances. 4) Detection of levels of oxidized proteins (Oxyblot analysis) in the presence of the substances. 5) Detection of Reactive Oxygen Species (ROS) in the presence of the substances. 6) Survival cellular ability under oxidative stress conditions in the presence of the substances. 7) Detection of chymotrypsin-like activity of the proteasome following incubation with the substances. 8) Detection of the levels of proteasome subunits following incubation with the substances. Whitening properties of the examined substances on melanocytes were evaluated by: A) Cellular whitening of melanocytes. B) Detection of tyrosinase expression, the main metalloglycoprotein that catalyzes several steps in the melanin pigment biosynthetic pathway.

This invention is pionneer since these substances have never been examined regarding their anti-aging or whitening properties. More specifically, advantage of biological aging markers is taken in order to define the biological action of the examined substances on human diploid fibroblasts. The results indicate that the anti-aging properties of the examined substances are the result of a combined action against cellular oxidation and maintenance of the proteasome activity, the main secondary anti-oxidant mechanism of defense. Oxidation and increase of ROS and consequently, increase of damaged and oxidized proteins is a hallmark of aging (K.B. Beckman and B.N. Ames (1998) The free radical theory of aging matures. Physiol. Rev. 78: 547-581). The proteasome is the main proteolytic mechanism of the cell responsible for the cellular detoxification and clearance by toxic factors like the oxidized proteins that accumulate with advanced age and accelerate the establishment of senescence (N. Chondrogianni and E.S. Gonos (2005) Proteasome dysfunction and mammalian aging: involved steps and factors. Exp. Gerontol. 40: 931-938). Our previous studies in human diploid fibroblasts have shown that proteasome assembly and function decreases with advanced age *in vivo* and *in vitro* and the appearance of senescence can be irreversibly induced upon proteasome inhibition (N. Chondrogianni, I. Petropoulos, C. Franceschi, B. Friguet and E.S. Gonos (2000) Fibroblast cultures from healthy centenarians have an active proteasome. Exp. Gerontol. 35: 721-728, N. Chondrogianni, F.L.L. Stratford, I.P. Trougakos, B. Friguet, A.J. Rivett and E.S. Gonos (2003) Central role of the proteasome in senescence and survival of human fibroblasts: induction of a senescence-like phenotype upon its inhibition and resistance to stress upon its activation. J. BioL Chem. 278: 28026-28037, N. Chondrogianni and E.S. Gonos (2004) Proteasome inhibition induces a senescence-like phenotype in primary human fibroblast cultures. Biogerontology 5: 55-61). On the other hand, we have also shown that overexpression of the proteasome leads to ameliorated response to oxidative stress whereas, it also confers lifespan extension, thus verifying that the proteasome is a longevity assurance mechanism (N. Chondrogianni, C. Tzavelas, A.J. Pemberton, I.P. Nezis, A.J. Rivett and E.S. Gonos (2005) Overexpression of proteasome beta 5 subunit increases the amount of assembled proteasome and confers ameliorated response to oxidative stress and higher survival rates. J. Biol. Chem. 280: 11840-118500). On top of that, we have also identified that centenarians, the best model of successful aging, possess an active proteasome, regardless their age (N. Chondrogianni, I. Petropoulos, C. Franceschi, B. Friguet and E.S. Gonos (2000) Fibroblast cultures from healthy centenarians have an active proteasome. Exp. Gerontol. 35: 721-728).

The aim of this invention is the identification of natural substances with anti-aging properties, mainly based on the activation and the maintenance of proteasome function and on a secondary base, based on the regulation of cellular oxidative load. Additional goals are the production of anti-aging products that due to their anti-oxidant properties and their rejuvenating effects could be used for as cosmetic products.

### ANTI-AGING PROPERTIES

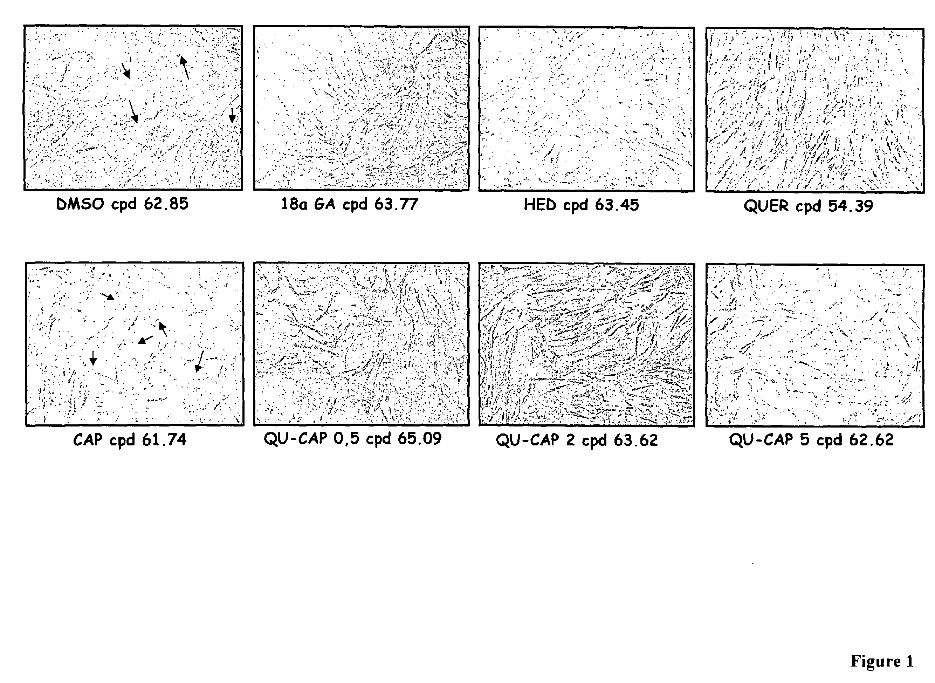

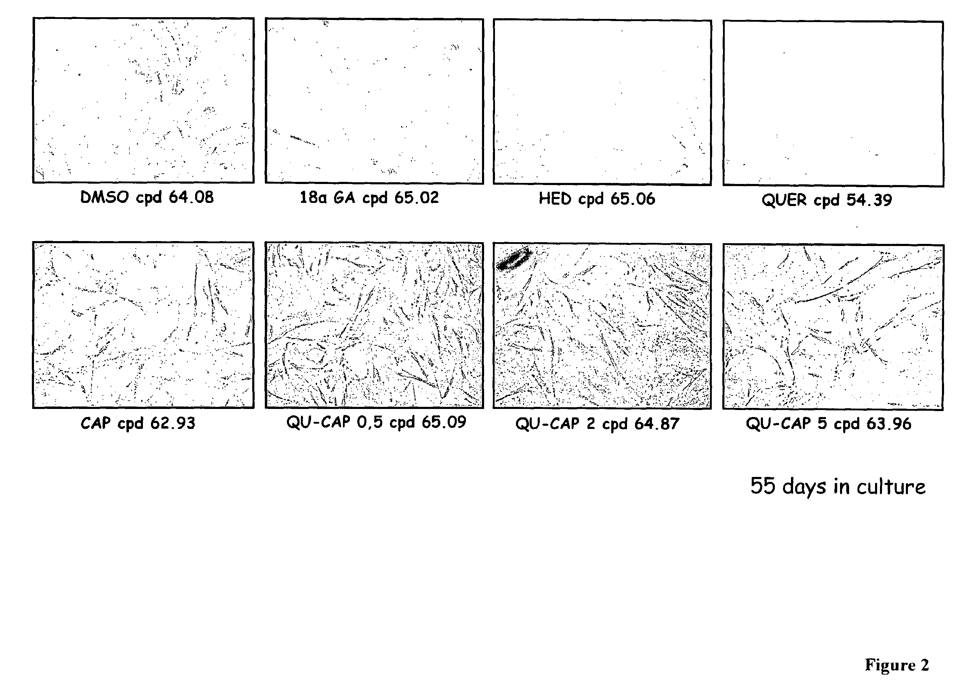

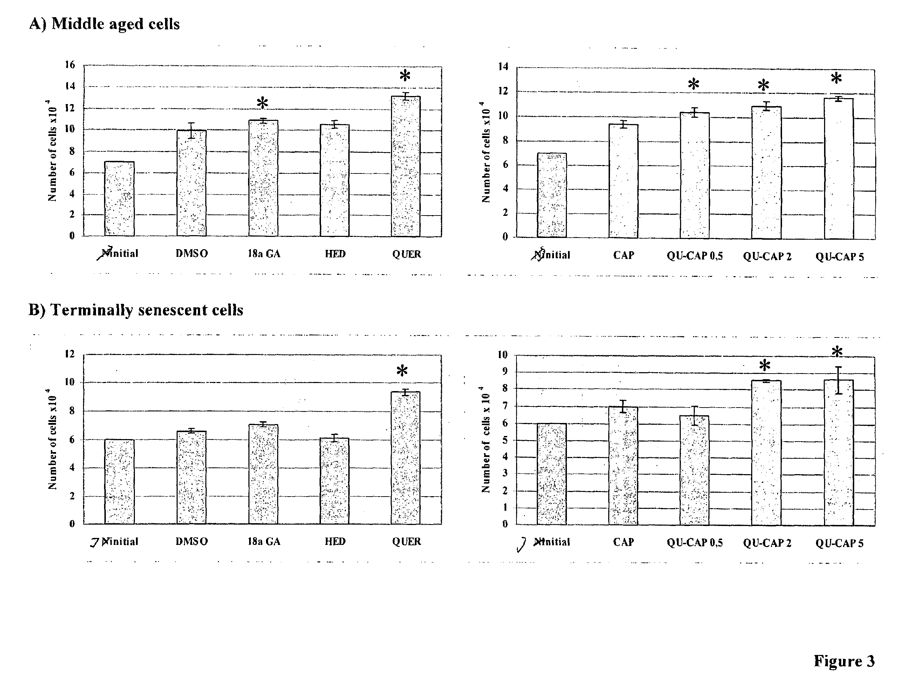

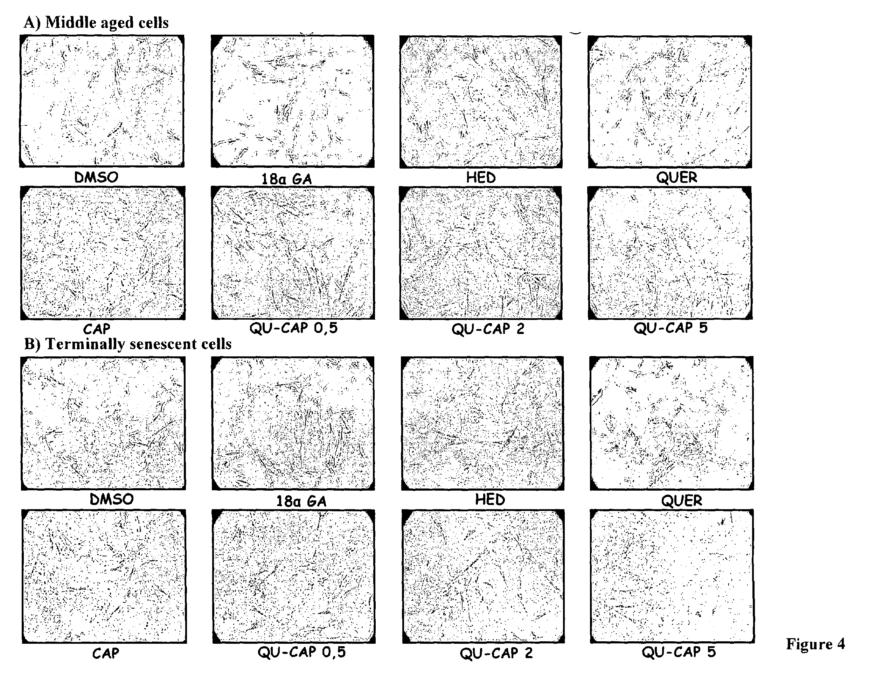

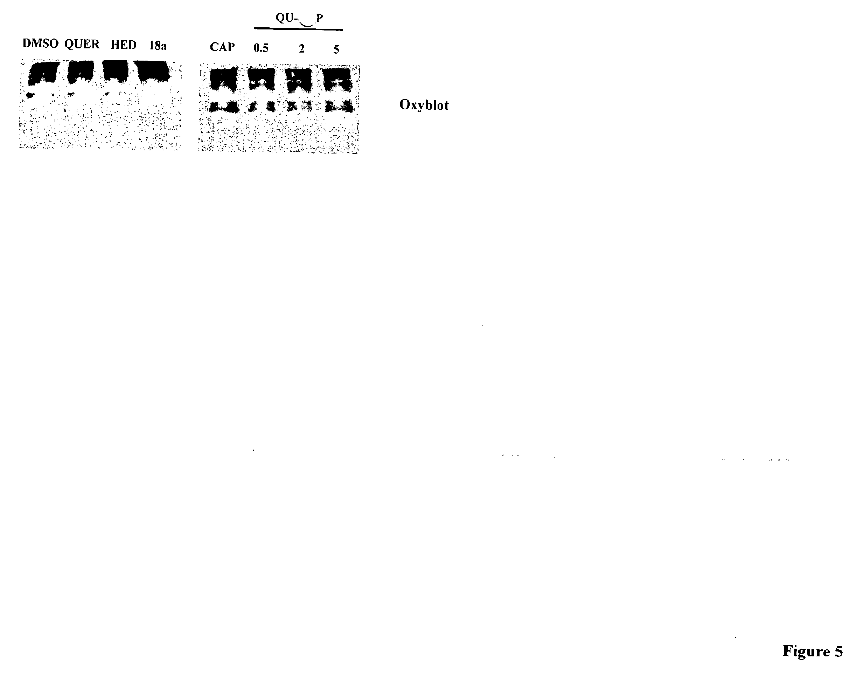

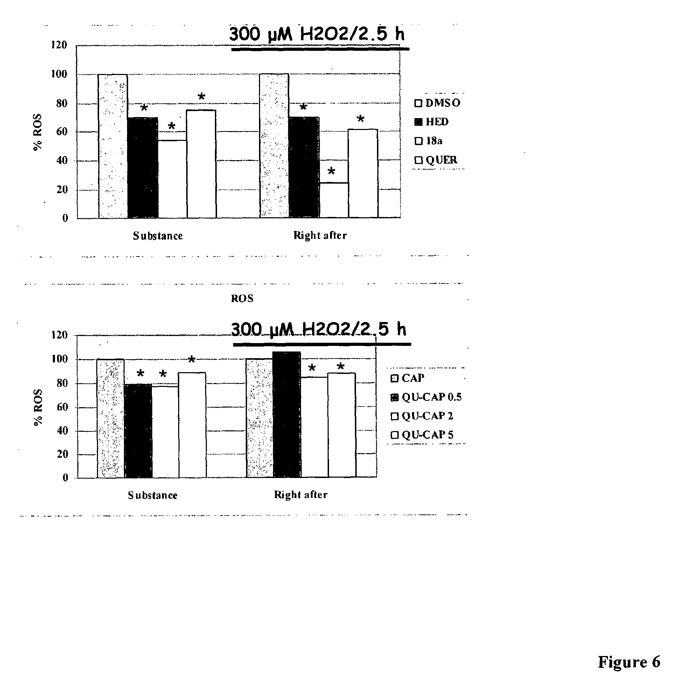

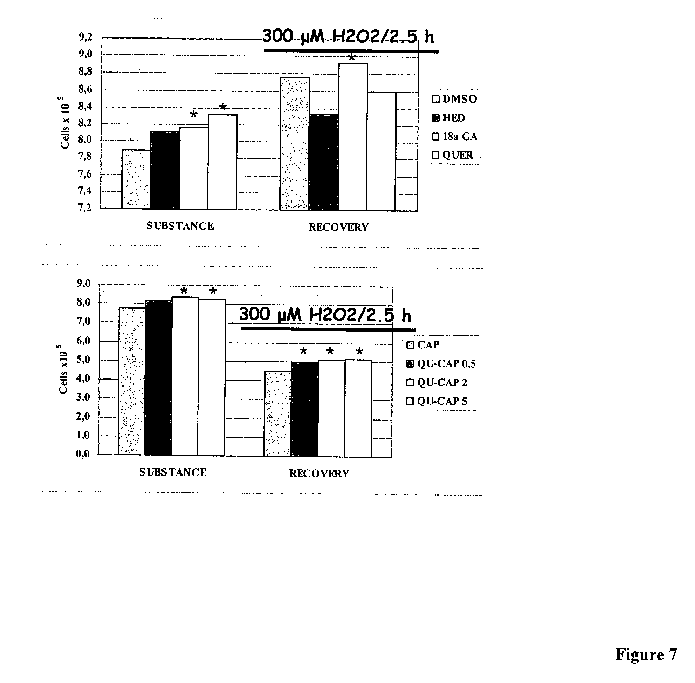

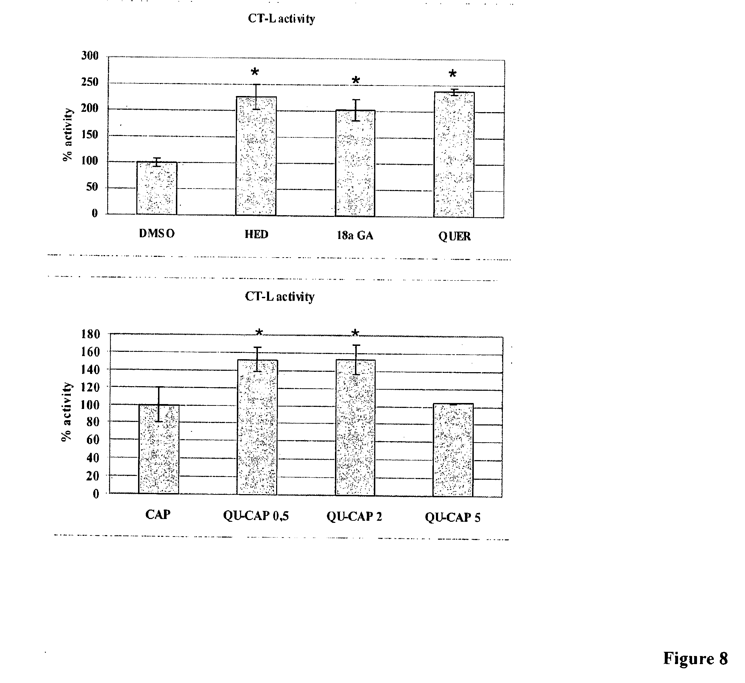

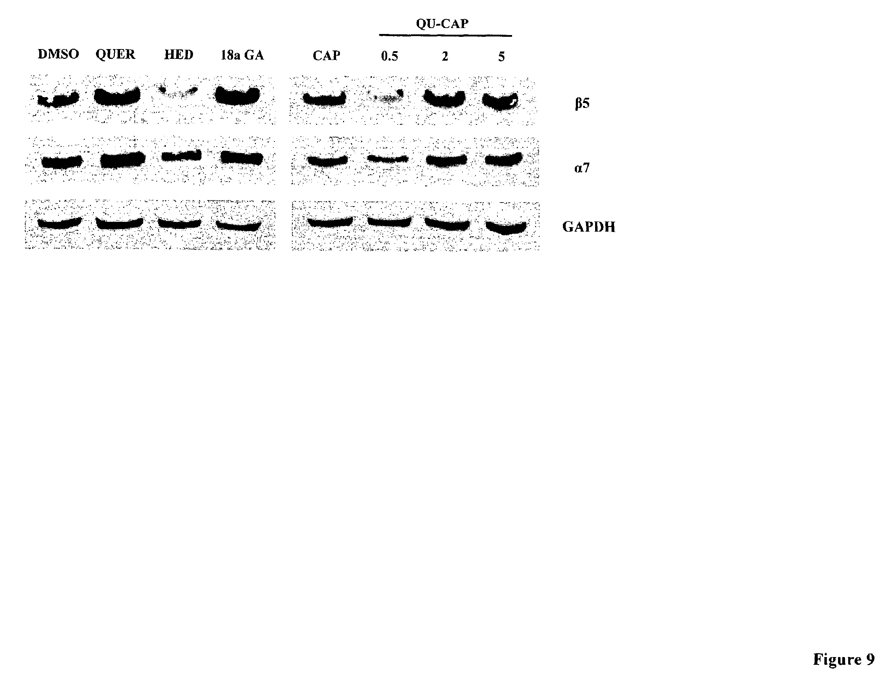

The steps, methods and results that were obtained regarding the anti-aging properties of the examined substances are the following:
**1) Study of the lifespan of human diploid fibroblasts *in vitro* in the presence of the substances.**
   **1.1.** HFL-1 human diploid fibroblasts (HDFs) were used in *in vitro* conditions (growth at 37°C, 5% CO₂ and 95% humidity).
   **1.2.** The cells were maintained in Dulbecco's modified Eagle's medium (DMEM ®; Invitrogen Life Technologies Inc.) supplemented with 10% (v/v) fetal calf serum, 100 Units/ml penicillin, 100 µg/ml strepromycin, 2 mM glutamine and 1% (v/v) non-essential amino acids (complete medium).
   **1.3.** The cells were constantly cultured in culture medium supplemented with the substances [final concentration for quercetin (QUER), 18α-glycyrrhetinic acid (18α GA), and hederagenin (HED): 2 µg/ml and for quercetin caprylate (QU-CAP) three concentrations were applied: 0.5, 2 and 5 µg/ml]. Quercetin, 18α-glycyrrhetinic acid and hederagenin were diluted in DMSO (dimethyl sulfoxide) whereas quercetin caprylate was diluted in caprilic/capric triglycerides (CAP). Therefore, the control cultures were incubated in medium supplemented with 0.1% DMSO or 0.1% CAP.
   **1.4.** The cells were replenished with fresh media supplemented with the substances or the diluents every 24 h and their numbers were examined using a Coulter Z2 ® counter (Coulter Corporation) until they reached senescence (approximately after 13 weeks). The cumulative population doublings performed by each culture were calculated using the following formula: CPD=Σ[PD] where PD=LOG[Nfinal/Ninitial]/LOG[2], where N final represents the number of cells that were measured when each culture reached confluence and N initial represents the number of cells that was initially seeded.
   **1.5.** Photographs of the cells maintained in cell culture and treated with the various substances for the same time period were taken.
   **1.6.** Table 1 shows the total number of cumulative population doublings (CPDs) performed by the different HFL-1 cultures that were treated with the various substances. More specifically, cells that were incubated with quercetin caprylate gave up to 2.8% more population doublings as compared to the population doublings performed by the relative control cultures. Cells that were incubated with the other substances gave up to 2.9% more population doublings as compared to their relative control cultures.
   **1.7.** Figure 1 shows the morphology of the cells treated with the various substances for the same time period. Cells that were treated with either of the tested substances maintained the young morphology for longer as compared to the control cultures (DMSO-treated and CAP-treated). Cells treated with the substances are more elongated and they grow in parralel arrays as compared to the control cultures that exhibit a higher number of flattened cells with more irregular shape. It is also worth noting that cells that were treated with the substances exhibited a younger phenotype as compared to their controls although they have performed more population doublings during the time that the photograph was taken.
**2) Detection of** β**-galactosidase activity.**
   **2.1.** HFL-1 HDFs were cultured as described in 1.1-1.4 for 10 consecutive weeks and were then subjected to β-galactosidase activity staining.
   **1.2.** Briefly, 10⁵ cells from each culture were seeded in 6-well plates. After 24 h cells were washed with PBS (phosphate buffered saline), fixed in 0.2% glutaraldehyde and 2% formaldehyde for 5 min, washed again with PBS, and finally stained for 24 h at 37 °C in the absence of CO₂, in staining solution (150 mM NaCl, 2 mM MgCl₂, 5 mM K₃Fe(CN)₆, 40 mM citric acid, and 12 mM sodium phosphate, pH 6.0) containing 1 mg/ml 5-bromo-4-chloro-3-indolyl-β-D-galactoside. Photographs were then taken.
   **1.3.** Figure 2 shows the β-gaiactosidase activity positivity of HFL-1 cultures treated with the various substances or the relative diluents for the same time period. Cells treated with the different substances appeared less positive to β-galactosidase activity staining (blue color) as compared to the relative control cultures where almost all cells appeared to be positive to the β-galctosidase activity staining, thus revealing maintenance of the young phenotype for longer. Furthermore, in full accordance with the photograph in Figure 1, the morphology of the cells verifies the above mentioned staining since cultures with less positive cells to β-galctosidase activity exhibited a younger morphology as compared to the rest cultures.
**3) Reversal of senescent phenotype by addition of the substances.**
   **3.1.** HFL-1 HDFs that have senesced *in vitro* were used (growth at 37°C, 5% CO₂ and 95% humidity). More specifically, middle aged HFL-1 HDFs (cpd 59) and terminally senescent HFL-1 HDFs (cpd 65) that had senesced in the absence of any substance were used.
   **3.2.** The cells were seeded and maintained in complete medium (see 1.2). Culture medium supplemented with the substances [final concentration for quercetin (QUER), 18α-glycyrrhetinic acid (18α GA), and hederagenin (HED): 2 µg/ml and for quercetin caprylate (QU-CAP) three concentrations were applied: 0.5, 2 and 5 µg/ml] or the diluents (0.1% DMSO or 0.1% CAP) were added 24 h later. The cells were replenished with fresh media supplemented with the substances or the diluents every 24 h for 1 week and their numbers were then examined using a Coulter Z2 ® counter (Coulter Corporation).
   **3.3.** Detection of β-galactosidase activity was performed in these cells as described above and photographs of the cells following β-galactosidase activity assay were taken.
   **3.4.** Figure 3 shows the number of (A) middle aged cells and (B) terminally senescent cells that were counted following I week treatment with the various substances. In middle aged cells, treatment with quercetin, 18α-glycyrrhetinic acid as well as with all three tested concentrations of quercetin caprylate led to higher proliferation rates as compared to the rates exhibited by the control cultures. In terminally senescent cells, treatment with quercetin or quercetin caprylate (2 and 5 µg/ml) induced a limited but sigmificant cell proliferation as compared to the absence of duplication in the control cultures whereas treatment with the rest of the substances did not show any significant differences.
   **3.5.** Figure 4 shows the morphology and the β-galactosidase activity positivity of (A) middle aged cells and (B) terminally senescent cells treated with the various substances for 1 week. In middle aged cells, treatment with quercetin, 18α-glycyrrhetinic acid and all three concentrations of quercetin caprylate, appeared to be less positive to β-galactosidase activity staining but however, since control cultures were not either heavily positive to β-gatactosidase activity staining, the main finding was that cells treated with these substances were induced to proliferate and furthermore, aquired a morphology more similar to the one exhibited by young cells (regular size, elongated cells, growth in parallel arays). In terminally senescent cells, treatment with quercetin and the higher concentrations of quercetin caprylate induced cell proliferation as mentioned above, while many of those cells were negative for β-gatactosidase activity. These cells were elongated and they acquired a morphology that exhibits more similarities with the morphology of young fibroblasts as compared to the relative control cultures where almost all cells were positive for β-gaiactosidase activity with a typical senescent phenotype (enlarged cells with accumulation of autofluorescent material, not growing in parallel arrays).
**4) Detection of levels of oxidized proteins (Oxyblot analysis) in the presence of the substances.**
   **4.1.** HFL-1 HDFs were cultured as described in 1.1- 1.4. Cells treated with the substances for 24 h and were then lysed and proteins were extracted and subjected to SDS-PAGE (sodium dodecylsulfate polyacrylamide gel electrophorese) followed by OxyBlot analysis that detects the carbonylated groups on proteins. OxyBlot^{™} is a protein oxidation detection kit.
   **4.2.** Figure 5 shows that cells treated with quercetin caprylate have less oxidized proteins as compared to the relative control cells. Regarding the other substances, cells treated with 18α-glycyrrhetinic acid exhibited slightly lower levels of oxidized carbonylated proteins as compared to the relative control cultures. However it is worth mentioning that oxyblot analysis detects only carbonylated groups on proteins which is just one oxidative stress-mediated post-translational protein modification. Therefore, this could account for the mild differences that this analysis reveals between treated cells and the relative control cultures.
**5) Detection of Reactive Oxygen Species (ROS) in the presence of the substances.**
   **5.1.** HFL-1 HDFs were seeded and maintained in complete medium (see 1.2). Culture medium supplemented with the substances [final concentration for quercetin (QUER), 18α-glycyrrhetinic acid (18α), and hederagenin (HED): 2 µg/ml and for quercetin caprylate (QU-CAP) three concentrations were applied: 0.5, 2 and 5 µg/ml] or the diluents (0.1% DMSO or 0.1% CAP) were added 24 h later and an additional incubation for 24 h followed. Cells were collected and detection of Reactive Oxygen Species (ROS) was performed in order to reveal ROS levels following the incubation with the substances. In replica cultures, culture medium supplemented with the substances as described above were also suplemented with 300 µM H₂O₂ for 2.5 h and cells were collected for detection of ROS following oxidative stress in the presence or absence of the substances.
   **5.2.** 2',7'-dichlorodihydrofluorescein diacetate (H2DCFDA, Molecular Probes, Invitrogen) was used for ROS detection. Cells (10⁵) were resuspended in prewarmed PBS ± the dye at a final concentration of 10 µM (loading buffer) and incubated at 37 °C for 30 min. Cells were then resuspended in prewarmed complete medium and incubated at 37 °C for 5 min. The absorption and the emission of the oxidation product were measured at 493 and 520 nm, respectively. Each sample was measured in quadruplicates.
   **5.3.** Figure 6 shows the percentages of ROS levels in cultures treated with the substances for 24h (Substance), in cultures treated with the substances for 24 h and being subjected to oxidative stress for 2.5 h (Right after) and in the relative control cultures (treated with 0.1 % DMSO or 0.1 % CAP). ROS levels of control cultures at each time point were arbitrary set to 100%. Cells treated with quercetin caprylate and quercetin without being subjected to oxidative stress (Substance) exhibited decrease of ROS levels ranging from 11% to 25% as compared to the relative control cultures. Cells treated with the other substances without being subjected to oxidative stress (Substance) exhibited decrease of ROS levels ranging from 30% to 46% as compared to the relative control cultures. When the cells were also challenged with 300 µM H₂O₂, treatment with quercetin caprylate or quercetin led to decrease of ROS levels by 12% to 39% while for treatment with the other substances the reduction of ROS levels was ranging from 30% to 75%.
**6) Survival cellular ability under oxidative stress conditions in the presence of the substances.**
   **6.1.** HFL-1 HDFs were cultured as described in 5.1. Following the incubation with the various substances cells were collected and counted using a Coulter Z2 ® counter (Coulter Corporation). In replica cultures, culture medium supplemented with the substances as described above were also suplemented with 300 µM H₂O₂ for 2.5 h. The cells were then thoroughly washed with PBS, supplemented with complete medium containing the examined substances and maintained in culture for additional 24 h. They were then collected and counted using a Coulter Z2 ® counter (Coulter Corporation).
   **6.2.** Figure 7 shows the total number of cells that survived after treatment with the various substances with or without being subjected to oxidative stress. Cells that were just treated with quercetin or quercetin caprylate exhibited higher survival rates ranging from 5.4% to 7.5% as compared to the cells that were treated with the diluents. For the rest of the substances, only treatment with 18α-glycyrrhetinic acid revealed a significantly increased survival rate that reached 3.5%. Additionally, cells that were treated with quercetin or quercetin caprylate and were then subjected to oxidative stress exhibited a higher survival rate reaching to 14.5% as compared to the survival rate of the relative control cultures. Regarding the rest substances, treatment with 18α-glycyrrhetinic acid during oxidative stress had a significant increase of survival that reached 2% as compared to the survival of the control cultures.
**7) Detection of chymotrypsin-like activity of the proteasome following incubation with the substances.**
   **7.1.** HFL-1 HDFs were cultured as described in 5.1. Following the incubation with the various substances for 24 h, cells were collected, lysed and proteasome activities assays were performed.
   **7.2.** Detection of the main proteasome activity (chymotrypsin-like; degrading after hydrophobic residues) was performed in crude extracts with the hydrolysis of the fluorogenic peptide LLVY-AMC ®, for 30 min at 37 °C. Proteasome activity was determined as the difference between the total activity of crude extracts and the remaining activity in the presence of 20 µM MG132 that is a specific inhibitor of the proteasome. Fluorescence was measured using a VersaFluor^{™} fluorescence spectrophotometer (BioRad Laboratories Inc.). Protein concentrations were determined using the Bradford method with BSA as a standard.
   **7.3.** Figure 8 shows that CT-L activity was induced by ∼1.5-2.5 folds in the presence of quercetin or quercetin caprylate and by ∼2-2.3 folds in the presence of the rest substances as compared to the activity detected in the relative control cultures.
**8) Detection of the levels of proteasome subunits following incubation with the substances.**
   **8.1.** HFL-1 HDFs were cultured as described in 5.1. Following the incubation with the various substances for 24 h, cells were collected, lysed and proteins were extracted and subjected to SDS-PAGE followed by immunoblot analysis against different proteasome subunits. Protein concentrations were determined using the Bradford method with BSA as a standard.
   **8.2.** Figure 9 shows the levels of α- and β-proteasome subunits in cells treated with the substances and their relative control cultures. In the presence of quercetin and quercetin caprylate elevated protein levels of both catalytic (β₅) and regulatory (α₇) proteasome subunits were revealed. The results were similar for 18α-glycyrrhetinic acid whereas for hederagenin we did not detect any up-regulation regarding proteasome subunits. In total, the increased amount of proteasome subunits expression levels provide an explanation regarding the increased proteasome activities recorded in these cells.

### WHITENING PROPERTIES

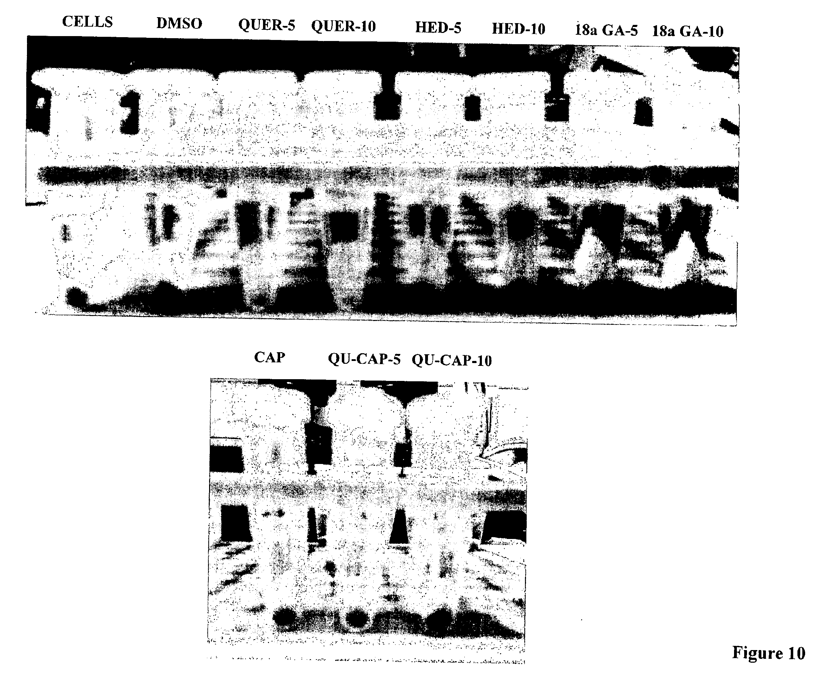

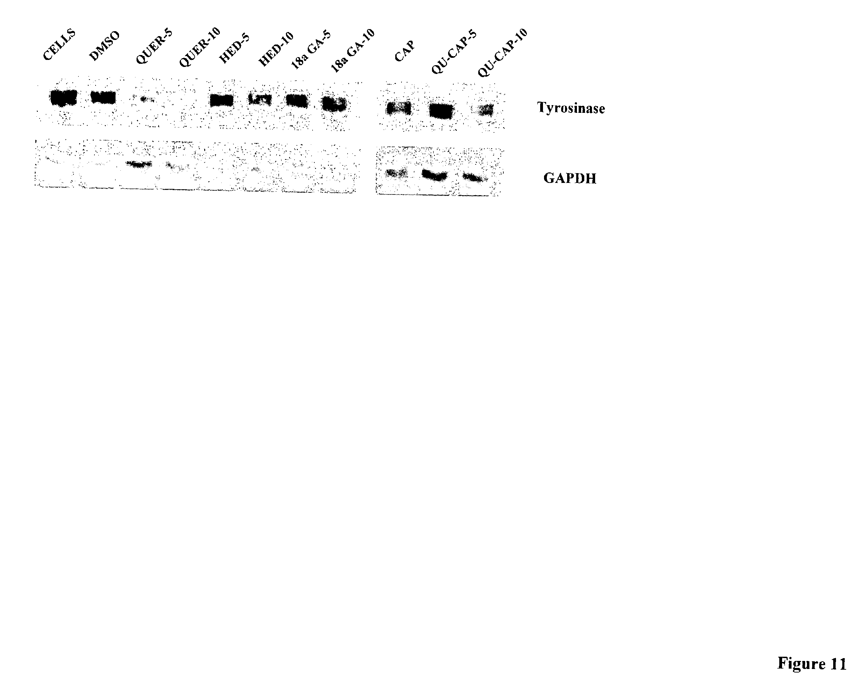

The steps, methods and results that were obtained regarding the whitening properties of the examined substances are the following:
**A) Cellular whitening of melanocytes.**
   **A.1.** Mouse melanocytes (B10F16 cell line) were used in *in vitro* conditions (growth at 37°C, 5% CO₂ and 95% humidity).
   **A.2.** The cells were maintained in Dulbecco's modified Eagle's medium (DMEM®; Invitrogen Life Technologies Inc.) supplemented with 10% (v/v) fetal calf serum, 100 Units/ml penicillin, 100 µg/ml strepromycin, 2 mM glutamine and 1% (v/v) non-essential amino acids (complete medium).
   **A.3.** 10⁵ cells were seeded in petri dishes and the following day they were fed with medium supplemented with the substances [final concentration for all substances: 5 µg/ml and 10 µg/ml]. Quercetin (QUER), 18α-glycyrrhetinic acid (18α GA) and hederagenin (HED) were diluted in DMSO whereas quercetin caprylate was diluted in caprilic/capric triglycerides (CAP). Therefore, the control cultures were incubated in medium supplemented with 0.1% DMSO or 0.1% CAP. The media were replenished every 24 h and the cells were kept under these conditions constantly for 72 h. Cells were then collected and pelleted and the cell pellet was photographed.
   **A.4.** Figure 10 shows the pellet of melanocytes treated either with the various substances or with the diluents. Treatment with both concentrations of quercetin and the lower concentration of hederagenin and 18α-glycyrrhetinic acid revealed whitening potential. However, the effect of quercetin was notably more intensed as compared to the one exhibited by the other substances. The result was positively correlated to the concentration of quercetin used.
**B) Detection of tyrosinase expression.**
   **B.1.** Mouse melanocytes were cultured as described in A.1.-A.3. After 72 h of treatment, the cells were collected, lysed and proteins were extracted and subjected to SDS-PAGE followed by immunoblot analysis against tyrosinase. Protein concentrations were determined using the Bradford method with BSA as a standard.
   **B.2.** Figure 11 shows the protein levels of tyrosinase in cells treated with the substances and their relative control cultures. In the presence of quercetin, quercetin caprylate and a high concentration of hederagenin reduced protein levels of tyrosinase were detected possibly giving an explanation on the whitening effect that was detected in these cells as already described in A.1-A.4.

### LEGENDS

### Table 1: Continuous treatment of HFL-1 cells with the substances extends their proliferative lifespan.

Number of cpds of HFL-1 cells treated with the substances or the relative diluents for 13 consecutive weeks. Continuous treatment of primary human fibroblasts with the substances extends their proliferative lifespan as compared to controls.

### Table 2: Amount of β-galactosidase positive cells in the culture.

### Figure 1: Continuous treatment of HFL-1 cells with the substances delays the appearance of the senescence phenotype.

Representative photographs of HFL-1 cells treated with the substances or the relative diluents after 8 weeks in culture. Continuous treatment of primary human fibroblasts with the substances retains the young morphology and delays the appearance of the senescence phenotype. Arrows indicate cells exhibiting typical senescent morphology.

### Figure 2: Continuous treatment of HFL-1 cells with the substances results to decreased β-galactosidase activity staining.

β-galactosidase activity staining (blue color) of HFL-1 cells treated with the substances or the relative diluents after 10 weeks in culture. Cells treated with the substances exhibited less positive cells to β-galactosidase activity staining as compared to the positive cells in the relative control cultures.

### Figure 3: Continuous treatment of middle aged and terminally senescent HFL-1 cells with the substances results to induction of proliferation.

Number of (A) middle aged cells and (B) terminally senescent cells that were constantly treated with the substances for 1 week and were then counted. Asterisks reveal statistically significant differences (p<0.05).

### Figure 4: Continuous treatment of middle aged and terminally senescent HFL-1 cells with the substances results to partial reversal of the senescent phenotype.

Morphology and β-galactosidase activity staining of (A) middle aged cells and (B) terminally senescent cells treated with the various substances for the 1 week. Arrows indicate cells negative to β-galactosidase activity staining.

### Figure 5: Treatment of HFL-1 cells with the substances results to decrease of intracellular oxidized proteins.

Oxyblot ® analysis in cells treated with the substances or the relative diluents after 24 h of treatment with the substances.

### Figure 6: Treatment of HFL-1 cells with the substances results to decreased intracellular ROS levels.

Percentages of ROS levels in cultures treated with the substances for 24 h (Substance), in cultures treated with the substances for 24 h and being subjected to oxidative stress for 2.5 h (Right after) and in the relative control cultures (treated with 0.1 % DMSO or 0.1% CAP). ROS levels of control cultures at each time point were arbitrary set to 100%.

### Figure 7: Treatment of HFL-1 cells with the substances results to increased survival rates.

Number of cells that survived after treatment with the various substances with or without being subjected to oxidative stress. Asterisks reveal statistically significant differences (p<0.05).

### Figure 8: Treatment of HFL-1 cells with the substances results to increased proteasome activities.

Percentage of CT-L activity in cells treated with the substances or the relative diluents after 24 h of treatment with the substances. Use of a proteasome inhibitor (MG132) in control reactions ensured the specificity of the enzymatic reactions. Mean value of activities was set at 100% in control cultures. Each column shows the average of three independent experiments, and error bars denote standard error.

### Figure 9: Treatment of HFL-1 cells with the substances results to increased protein expression levels of proteasome subunits.

Immunoblot analysis of representative subunits of 20S complex, β-type subunits (β₅, 22.9 kDa), and α-type subunits (α₇, 28.4 kDa) in cells treated with the substances or the relative diluents. Equal protein loading was verified by stripping the membranes and reprobing them with GAPDH (37 kDa) antibody (bottom panels).

### Figure 10: Treatment of mouse melanocytes with the substances results to cellular whitening.

Photographs of pellet of melanocytes that were treated with the substances for 72 h.

### Figure 11: Treatment of mouse melanocytes with the substances results to decreased levels of tyrosinase.

Immunoblot analysis of tyrosinase (70-80 kDa) in melanocytes treated with the substances or the relative diluents. Equal protein loading was verified by stripping the membrane and reprobing them with GAPDH (37 kDa) antibody (bottom panel).

### The results of the above studies can be summarized as follows:

### 1. Study of the lifespan of human diploid fibroblasts in vitro in the presence of the substances.

As described in sections 1 and 2, cells that were treated with quercetin, quercetin caprylate and 18α-glycyrrhetinic acid exhibited a delay in the appearance of the senescent phenotype. This was accompanied by all the aspects that characterize such a delay, i.e. retention of young morphology, regular shape, lower amount of β-galactosidase positive cells.

### 2. Reversal of the senescent phenotype/Rejuvenation effect

As described in section 3, middle aged cells or terminally senescent cells that were treated with quercetin, quercetin caprylate and 18α-glycyrrhetinic acid, regained a younger morphology and were induced to limited but significant proliferation.

### 3. Decrease of the intracellular oxidative load

As described in sections 4-6, cells that were treted with quercetin caprylate and 18α-glycyrrhetinic acid possessed lower amounts of oxidized proteins as compared to the relative control cultures. All cultures treated with the tested substances had lower intracellular ROS levels as compared to the control cultures. Furthermore, cells treated with quercetin, quercetin caprylate and 18α-glycyrrhetinic acid survived better to an oxidative stress challenge as compared to the control cells.

### 4. Activation of the proteasome.

As described in sections 7 and 8, treatment with all the tested substances induced an activation of the chymotrypsin-like activity of the proteasome and an increased expression of the proteasome subunits. Consequently, cells treated with these substances possessed enhanced secondary anti-oxidant mechanisms.

### 5. Cell whitening.

As described in sections A and B, treatment of melanocytes with quercetin, hederagenin and 18α-glycyrrhetinic acid revealed whitening potential that was accompanied by a decrease of the protein expression levels of tyrosinase, the main metalloglycoprotein that catalyzes several steps in the melanin pigment biosynthetic pathway that is mainly responible for the age spots.

**Table 1**

| **Substance** | **cpd** |
|---|---|
| **DMSO** | 65.1 |
| **18α glycyrrhetinic acid** | 67 (+2.9%) |
| **Hederagenin** | 65.4 (+0.5%) |
| **Quercetin** | 57.24 |
| **Caprilic/Capric Triglycerides** | 64.2 |
| **Quercetin caprylate 0, 5 µg/ml** | 65.9 (+2.6%) |
| **Quercetin caprylate 2 µg/ml** | 66.0 (+2.8%) |
| **Quercetin caprylate 5 µg/ml** | 65.4 (+1.9%) |

**Table 2**

| **Substance** | **cpd** | **% β-galactosidase positive cells** |
|---|---|---|
| DMSO | 64.08 | 77 |
| 18α glycyrrhetinic acid | 65.02 | 37.7 |
| Hederagenin | 65.06 | 31.8 |
| Quercetin | 54.39 | 13.5 |
| Caprilic/Capric Triglycerides | 62.93 | 75 |
| Quercetin caprylate 0,5 µg/ml | 65.09 | 36.6 |
| Quercetin caprylate 2 µg/ml | 64.87 | 32.3 |
| Quercetin caprylate 5 µg/ml | 63.96 | 47 |

55 days in culture

## Claims

1. Use of quercetin, 18a-glycyrrhenitic acid and/or hederagenin and/or its derivatives in a composition for the treatment of skin having anti-aging and/or whitening properties.

2. Use of quercetin, quercetin caprylate, 18a-glycyrrhenitic acid and/or hederagenin and/or its derivatives in a composition for improving the morphology of diploid fibroblasts and for promoting whitening of melanocytes cells in human tissue.

3. Use according to claim 2, **characterized in that** the improvement comprises an increase of the proliferation of diploid fibroblasts during senescence.

4. Use according to claim 2 or 3, **characterized in that** the improvement comprises a delay and/or reversal of the senescence of diploid fibroblasts.

5. Use according to anyone of claims 2 to 4, **characterized in that** the improvement comprises an improved capability of resisting against oxidative stress.

6. Use according to anyone of claim 2 to 5, **characterized in that** the improvement comprises an activation of proteasome.

7. Use according to claim 1 or 2, **characterized in that** the quercetin derivative is quercetin caprylate or a glycoside of quercetin.

8. A method for testing the anti-aging properties of a compound for the treatment of skin, **characterized in that** the compound is added to a culture of diploid fibroblasts.

9. The method of claim 8, **characterized in that** the number of diploid fibroblasts is counted and the performed cumulative population doublings are calculated after treatment with the compound for a given period of time.

10. The method of claim 8, **characterized in that** the morphology of the diploid fibroblasts of the culture is determined after treatment with the compound for a given period of time.

11. The method of claim 8, **characterized in that** the β-galactosidase activity of the culture is determined.

12. The method of claim 8, **characterized in that** a culture with senescent diploid fibroblasts is used and the reversal of the senescent phenotype is determined after treatment with the compound for a given period of time.

13. The method of claim 8, **characterized in that** the level of oxidized proteins of the cultured diploid fibroblasts is determined after treatment with the compound.

14. The method of claim 8, **characterized in that** the level of reactive oxygen species of the cultured diploid fibroblasts is determined after treatment with the compound.

15. The method of claim 8, **characterized in that** the number of diploid fibroblasts of the culture is counted after treatment with the compound and in the presence of an oxidizing agent for a given period of time.

16. The method of claim 8, **characterized in that** the chymotrypsin like activity of the proteasome is determined after treatment of the culture of diploid fibroblasts with the compound for a given period of time.

17. The method of claim 8, **characterized in that** the protein expression levels of proteasome subunits is determined after treatment with the compound for a given period of time.

18. A method for testing the whitening properties of a compound for the treatment of skin, **characterized in that** the compound is applied to a culture of melanocytes and/or the tyrosinase expression is determined.
